# EUROPEAN PATENT APPLICATION

(11) **EP 1 790 215 A1**
(43) Date of publication of application: **30.05.2007**
(21) Application number: 05078007.1
(22) Date of filing: 27.12.2005
(51) Int. Cl.: A01H 3/04, A01G 7/06, A01N 43/90

(54) **Improved orchid culturing method**

(30) Priority: 25.11.2005 EP 05077680; 02.12.2005 NL 1030581
(71) Applicant: Bakker, Joost Petrus Jacobus, 2377 CA Oude Wetering (NL)
(72) Inventor: Bakker, Joost Petrus Jacobus, 2377 CA Oude Wetering (NL)
(74) Representative: Winckels, Johannes Hubertus F.

(57) **Abstract**

The invention describes a method to increase the number of inflorescences and/or minimise the need for a cold period for the induction of an inflorescence in an orchid by administration of a cytokinin, preferably 6-BAP, to said orchid, preferably wherein said orchid has a monopodial growth, more preferably wherein said orchid is of the genus *Phalaenopsis.* Further part of the invention are plants which have more than two inflorescences.

## Description

The invention relates to an improved method for the culturing of orchids, more specifically *Phalaenopsis.*

The sales of potted orchids has increased steadily over the last 30 years. In 2000, wholesale orchid sales were approximately $100,000,000, of which about 75% was accounted for by *Phalaenopsis* (Griesbach, R.J., 2002. Development of Phalaenopsis Orchids for the Mass Market. p. 458-465, In: Janick J. and Whipkey A. (eds), Trends in new crops and new uses, ASHS Press, Alexandra, VA).

*Phalaenopsis,* or the moth or butterfly orchid, is a genus of the family of Orchidiaceae, which contains at least 60 different species. However, in the recent past many varieties and hybrids have been produced for commercial purposes, resulting in a large assortment of pot flowers.

All orchids have these five basic features :
- the presence of a column
- the flower is bilaterally symmetrical
- the pollen are glued together into the pollinia, a mass of waxy pollen on filaments.
- the seeds are microscopically small, lacking endosperm (food reserves).
- the seeds can, under natural circumstances, only germinate in symbiosis with specialized fungi. Under artificial circumstances, however, germination is possible "in vitro" on sterile substrates or agar in specialized laboratories.

*Phalaenopsis* shows a monopodial growth habit. An erect growing rhizome produces from the top one or two alternate, thick and fleshy, elleptical leaves a year. The older, basal leaves drop off at the same rate. The plant retains in this way four to five leaves. They have no pseudobulbs. The raceme (flowering stem or stalk, spike or inflorescence) appears from the stem between the leaves. *Phalaenopsis* typically produces a single raceme, which consists of up to 25 flowers. The species and hybrids of *Phalaenopsis* that bloom in later winter or early spring require a period of about six weeks of exposure from 25°C-30°C (culture) to 15°C-20°C (induction) to trigger the emergence of the inflorescence (Report "Cultivation Guidelines Phalaenopsis Pot Plant. Anthura B.V. and Bureau IMAC Bleiswijk B.V., http://www.anthura.nl). There is an absolute requirement for the presence of light while plants are exposed to the proper temperatures for spiking (= emergence of the inflorescence). Although the plants are marketed year round, winter is an important selling season. This means that for the plants which have to be sold during winter, the cold induction has to be performed during the summer months. This means that the greenhouses need to be cooled down to at least 20 °C, which increases the costs of growing the plants. It would be very advantageous if this cooling period could be abolished or diminished.

It is also desirable to generate more inflorescences on the same plant, since this would yield more flowers per plant, and thus would increase its value. Depending on the length and temperature of the cold induction *Phal;aenopsis* plants normally induce one or two flower stalks

Thus, there is still need for a method to either abolish or diminish the cold induction period and/or to induce multiple raceme formation in orchids.

### SUMMARY OF THE INVENTION

The present inventors now have found a method to increase the number of inflorescences and/or to diminish the need for a cold period for the induction of an inflorescence in an orchid by administration of a cytokinin to said orchid, preferably wherein said orchid has a monopodial growth, more preferably wherein said orchid is of the genus *Phalaenopsis.* Said cytokinin is preferably 6-BAP (6-benzylaminopurine), which can be applied in a concentration in the range of about 2 mg to about 30 mg per plant, preferably about 5 mg to about 26 mg per plant, more preferably about 10 mg to about 24 mg per plant, most preferably about 20 mg per plant. In aqueous spraying solution, this would result in a range of about 100 ppm to about 1500 ppm, preferably about 250 ppm to about 1300 ppm, more preferably about 500 ppm to about 1200 ppm, most preferably about 1000 ppm.

A preferred embodiment of the invention is wherein the administration of the cytokinin is done by spraying a solution comprising said cytokinin.

Additionally, for better uptake of the cytokinin, the orchid is kept in the dark for a period of about 6 to about 14 hours after spraying. A similar effect, enhanced uptake of cytokinine, can be achieved by increasing the relative humidity of the air in the greenhouse. These measures can be taken separately, dependant on the climatic conditions inside and outside the greenhouse. Also for better uptake the spraying solution can additional comprise a wetting agent, preferably Zipper®, and/or a co-solvent, preferably DMSO.

A further preferred embodiment comprises the administration of a rooting hormone, preferably **NAA** to the orchid (uptake of NAA takes place both ath the leaves and at the roots). Administration of the rooting hormone preferably takes place at transfer of the young plants to pots and can be performed to about 4 weeks previous to the cold induction period.

A following embodiment of the invention is an orchid, preferably an orchid with a monopodial growth, more preferably an orchid of the genus *Phalaenopsis,* which has more than two inflorescences, alternatively more than 3 inflorescences, or alternatively more than four inflorescences. Also a group of orchids of five or more plants of the same variety, having an average number of inflorescences of more than 2.9, preferably more than 2.5, more preferably more than 2.1, is included in the scope of the present invention. Also contemplated are the use of a cytokinin to induce the forming of adventitious inflorescences in an orchid and the use of a cyokinin to abolish or diminish the need for a cold period to induce inflorescences.

### DESCRIPTION OF THE DRAWINGS

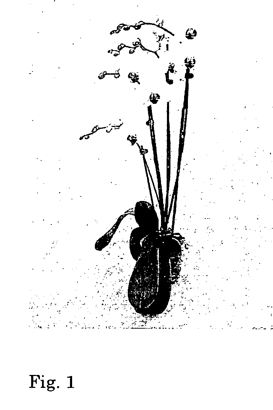

Fig. 1 shows a plant of the species *Phalaenopsis Hybrid* showing four flower stalks.

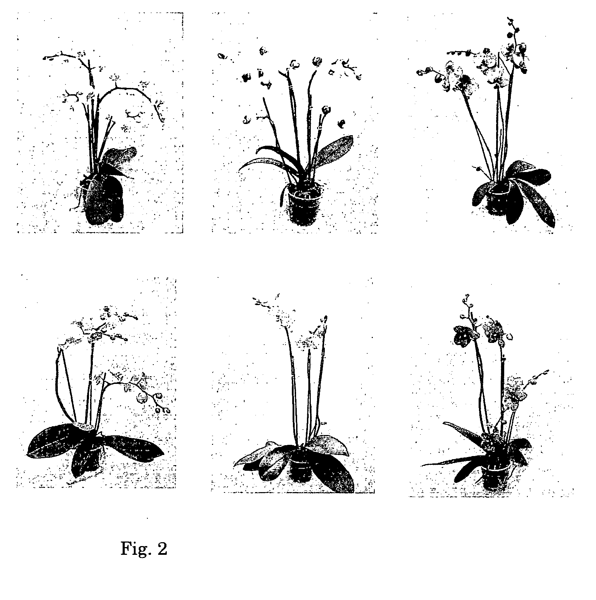

Fig. 2 shows results of example 1.

### DETAILED DESCRIPTION OF THE INVENTION

Cytokinins have the effect of increasing the mitosis (cell division) of shoot and buds. Some cytokinins increase the size or number of the plant cells and e.g. makes leaves larger. The cell differentiation can be controlled (e.g. for production of calli). Growth of roots is inhibited. Cytokinins are adenine derivatives and kinetin can e.g. be synthesised from heating nucleic acid. Known cytokinins are: 2iP N6-(3-methylbut-2-enyl)adenine, Kinetin (6-furfurylaminopurine), Benzyladenine (N6-benzyladenine), BA or BAP (6-benzylaminopurine), Zeatin, Adenine sulfate (2C₅H₅N₅·H₂SO₄) and Thidiazurone (1-phenyl-3-(1,2,3-tiadiazole-5-yl)urea). Cytokinins are heavily used in orchid production during tissue culture, to induce shoot regeneration. Further, it has been demonstrated that cytokinins can be used to induce keiki's in orchids (a keiki is a small plant which grows from one of the nodes along the (flower-bearing) stem). Usually these keiki's are introduced by applying cytokinines (commercially available as 'keiki paste', e.g. KeikiGrow from Plant Hormones Canada) after flowering. However, it has been reported (Wang, Y-T.: Medium, Nutrition and Flower Induction in Potting Blooming Orchids, ASHS-2000 Symposium, http://primera.tamu.edu/orchids/wang.htm) that application of cytokinin (BA or a combination of PBA and GA), whereas this was shown to trigger spiking - by application to the mature pseudobulbs, but not the developing ones - in *Dendrobium,* was unable to induce off-season spiking in *Phalaenopsis.* One possible reason for this failure is that the development of the inflorescence in *Dendrobium* occurs through so-called pseudo-bulbs, which are absent in *Phalaenopsis.*

The inventors, however, have now found that application of a cytokinin yields the emergence of adventitious inflorescences in orchids, especially orchids with a monopodial growth, preferably orchids that do not have pseudobulbs, and morepreferably *Phalaenopsis,* and/or reduces the need for a cold induction period. Upon cytokinin application it appears that sometimes even up to four or more additional flowers stalks appear. These additional spikes are similar to the original one and can also bear a large amount of flowers (under optimal growing conditions).

The cultivation and production of potted orchids in general and specifically *Phalaenopsis,* nowadays has taken an enormous leap due to the availability of tissue engineering for large scale production of clones. Since the 1970's several tissue culture protocols specific for *Phalaenopsis* have been developed. These tissue explants (mostly explants from the flower stalk) are regenerated to plantlets in large facilities, which have capacities of producing millions of plantlets a year. It is also possible to cultivate the orchids from seed, which would yield a pottable plant after about 15 months. The grower obtains these small plantlets and cultivates them further (for at least another 20 weeks) before induction of the florescence is initiated. After about 6 weeks at lower temperature, it will take about another 16 weeks to grow the plant, now bearing a flower stalk, into a sellable product, which is a plant which has just started flowering. The cultivation from plantlet to flowering plant is almost exclusively done in greenhouses because of the requirements of temperature control.

Normally the plants are held at a temperature of about 25 °C to about 30 °C, preferably about 27 °C. When flowering needs to be induced the temperature is lowered to about 15 °C to about 20 °C, preferably about 18 °C. In summer, in temperate climates, this would approximate the outside temperature, which means that this cold period can be achieved by only terminating the previous heating to 27 °C, whereupon the temperature in the greenhouse would drop to the outside temperature. However, since this outside temperature is far from constant and - in hot summers - would easily be above the desired temperature of max 20 °C, many breeders opt for active cooling.

It has now appeared possible to minimise the active cooling and still be able to induce the inflorescence by applying cytokinin. This treatment not only minimises the cold induction, but it appeared that more flower stalks are formed than with the normal cold induction. Whereas plants which have been cold induced normally would develop only one flower stalk (or two), treatment with cytokinin normally would give two flower stalks en in many cases more than two.

Preferably synthetic cytokinines are used, most preferably BAP because of its low cost: purification of naturally occurring cytokinines is time consuming and expensive. Further it is know that synthetic cytokininies are not toxic in the concentrations used, nor for plants nor for animals, including humans (TAP Report for 6-benzyladenine, January 2004, at http://www.ams.usda.gov/nop/NationalList/6BenzyladenineFinalnoCBI.pdf). Synthetic cytokinines according to the present invention are substances that show a cytokinine activity en are not to be found as such in nature, notably in plants (e.g. thidiazuron, benzyladenine and 6-benzylaminopurine).

Application of the cytokinin can be performed in any conventional way, but in greenhouses with many plants, it is preferable to apply the cytokinin by spraying with an aqueous solution. The concentration of the cytokinin to be sprayed can vary, but should be in the range of about 100 ppm to about 1500 ppm, preferably about 250 ppm to about 1300ppm, more preferably about 500 ppm to about 1200 ppm, most preferably about 1000 ppm.

Spraying is done with an amount of about 100 liters of spraying solution for an amount of approximately 20.000 plants. Thus, each plant will receive about 1/200 liter, or 5 ml of spraying solution. Spraying can be performed using standard spraying equipment, such as 5- 25 Bar pressurepump with single or multiple nozzles as commonly used for application of fungicides and/or pesticides. Such equipment is commercially available. This equipment preferably nebulises the spraying solution, in order that both upper and under surface of the leaves come into contact with the spraying solution.

When recalculating the above mention preferred concentrations in amounts of cytokinine applied to individual plants, it would result in about 2 to about 30 mg per plant, preferably about 5 mg to about 26 mg per plant, more preferably 10 mg to about 24 mg per plant, most preferably about 20 mg per plant.

Next to administration via spraying solution, the cytokinine can also be applied according to other ways known in the art, e.g. through powdercoating, electrospray, through normal techniques for watering (e.g. ebb- and floodtechnique) or through direct application on or next to the plant, e.g. in the form of pastes, patches or strips, or plantsticks with slow release of the cytokinin to the substrate.

To increase the uptake of the cytokinin by the plants additives, such as co-solvents or wetting agents, can be added to the cytokinin solution. One co-solvent which is preferably used in the invention is DMSO, which preferably is added to the spraying solution to a final concentration of 1 liter per 100 liter spraying solution. As wetting agent the commercially available Zipper® (an organically modified trisiloxane, obtainable from Asepta, Delft., The Netherlands) can be used in a final concentration of 10-500 ml per 100 liter spraying solution, preferably 100 ml/liter.

Generally all means that enhance the uptake of the cytokinin by the plant can be used. Next to the above discussed wetting agent Zipper®, other compounds that decrease the surface tension of the liquid are equally applicable. Also agnets that enhance the penetration of the cytokinin, such as DMSO (Broome, O.C., Zimmerman, R.H., (1976) J. Amer. Soc. Hort. Sci. 101:28-30) can be useful in the present invention.

To further increase uptake of the cytokinin by the plants, the plants can be covered from the light source, directly after spraying, e.g. by a plastic tent. By thus inducing an artificial night environment the stomata of the plants will open, which facilitates uptake. Further, because of the low light level, evaporation of the spraying solution will minimalise, which means that the sprayed solution will stay in contact with plants for a longer time. Further, in the case of a tent, ventilation will be reduced, which decreases evaporation and increases the relative humidity of the air, which also enhances uptake.

Preferably, covering of the plants only lasts for about 4-48 hours, since orchids need light to grow. After removal of the cover, plants are allowed to continue to grow at the low temperature conditions for another three to seven weeks, preferably about five weeks. After that, the temperature regimen should be returned to the high temperature, i.e. preferably between about 18 °C and 25 °C, to develop the plants to marketable early flowering plants for another 8-23 weeks. If desired, plants can be 'harvested' already earlier and transported or marketed.

Growing conditions for the plants throughout the whole greenhouse stay are the same, except for the above discussed variations in light and temperature. Preferably this means that the plants are potted in pots with a diameter of 10-20 cm, with a growing medium of bark, coconut, Styrofoam, sphagnum moss and turf. Preferably they are stored on table-high racks, which facilitates handling. Light intensity on the surface of the pot is 3000-15000 lux (see Schapendonk, A.H.C.M., "Belichting Phalaenopsis", PT-Project 12170, productverslag, Productschap Tuinbouw). Normal daylight is maintained, but preferably in wintertime a lights on:lights off regime of 14:10 hours is used. Watering is preferably automated and is taken care of by a sprinkler system or any other spraying system, an ebb and flow system, via gutters running along the racks, or by submerging the pots in a water basin Nutrients and/or fertiliser are added to the water on a regular basis (EC 0,5 - 2, NPK 20-20-20). See also ook "Cultivation Guidelines Phalaenopsis Pot Plants, *supra.*

A preferred embodiment of the invention is the additional application of hormones which promote root growth, such as commercially available rooting hormone products. To ensure optimal uptake of water and nutrients, a well-developed root system is essential, especially when more than one inflorescences need to be supported. Therefore, it is preferred to add rooting hormone substances to the water and nutrients to induce extra root growth to be able to support the extra inflorescences. Preferably, the rooting hormone is given when transplanting the pottable plants until 4 weeks prior to cooling for induction.

### EXAMPLES

### Example 1

In June 2004 three groups of *Phalaenopsis* plants have been induced 6 months after potting. For testing 100 plants were treated and 100 non-treated controls were used per variety. Both groups of plants were cultured as described above.Induction of spiking started at week 28 by spraying a solution of 1000ppm 6-BAP (BAP-10, Plantwise, Louisville, Kentucky USA), 0.1% Zipper, 1%DMSO] to the treated plants, while the controls did not receive this treatment. The results in Table 1 show the number of inflorescences per plant for the different testgroups.

**Table 1**

| Variety | Average stemcount untreated | Average stemcount treated |
|---|---|---|
| Dutch Lady | 1,58 | 3,84 |
| Maliby Leopard | 1,51 | 3,12 |
| Anthura Malaga | 1,23 | 3,61 |
| Lippe Flair 344 | 1,32 | 3,38 |
| Golden Treasure | 1,44 | 2,97 |
| Brother John Red Delight | 1,13 | 3,20 |

From this experiemnt it appears that groups of orchids of at least 5 plants per variety, or alternative 10, 20, 30, 40, 50 or 100 plants, can be produced with a method as described above, which have on average more tah 2 inflorescences. To discriminate such a group from a random group of five or more control plants of one variety, it can be stated that the group of treated plants has at least an average of 2.9 or more, preferably 2.5 or more and more preferably 2.1 or more flowering stalks. These racemes can support flowering flowers or not.

## Claims

1. Method to increase the number of inflorescences and/or to minimise the need for a cold period for the induction of an inflorescence in an orchid by administration of a cytokinin to said orchid, preferably wherein said orchid has a monopodial growth or wherein said orchid does not form pseudobulbs, more preferably wherein said orchid is of the genus *Phalaenopsis.*

2. Method according to claim 1, wherein said cytokinin is a synthetic cytokinin, preferably 6-BAP (6-benzylaminopurine).

3. Method according to claim 1 or 2 wherein the amount of administered cytokinin per plant is in the range of about 2 mg to about 30 mg per plant, preferably about 5 mg to about 26 mg per plant, more preferably about 10 mg to about 24 mg per plant, most preferably about 20 mg per plant.

4. Method according to one of the previous claims, wherein the concentration of the cytokinin in aqueous solution is in the range of about 100 ppm to about 1500 ppm, preferably about 250 ppm to about 1300 ppm, more preferably about 500 ppm to about 1200 ppm, most preferably about 100 ppm.

5. Method according to any of the previous claims, wherein the administration of the cytokinin is done by spraying a solution comprising said cytokinin.

6. Method according to one of the previous claims, wherein the orchid after spraying is kept in the dark for a period of about 6 to about 14 hours.

7. Method according to claim 4 or claim 5, wherein the spraying solution additional comprises a wetting agent, preferably Zipper®, and/or a co-solvent, preferably DMSO.

8. Method according to any of the previous claims wherein a rooting hormone, preferably NAA is applied to the roots of the orchid prior to the administration of the cytokinin.

9. Method for producing an orchid with more than two inflorescences by treating said orchid with a method according to any of the conclusions 1-8.

10. Method for producing a group orchids of 5 or more plants with an average number of flowering stalks of 2.9 or more, preferably 2.5 or more and more preferably 2.1 or more by treatment of said group of orchids with a method according to any of the conclusions 1-8.

11. Group of orchids of the same variety of 5 or more plants, which have an average amount of flowering stalks of 2.9 or more, preferably 2.5 or more and more preferably 2.1 or more.

12. Orchid, preferably an orchid with a monopodial growth, more preferably a plant of the genus *Phalaenopsis,* which has more than two inflorescences.

13. Use of a cytokinin to induce the forming of adventitious inflorescences in an orchid.

14. Use of a cyokinin to minimise the need for a cold period to induce inflorescences.

15. Orchid that has been treated according to a method according to any of claims 1-8, wherein the cytokinine is a synthetic cytokinin.

16. Orchid that comprises a synthetic cytokinin.
